# EUROPEAN PATENT SPECIFICATION

(11) **EP 3 688 219 B1**
(45) Date of publication and mention of the grant of the patent: **23.07.2025**
(21) Application number: 18801009.4
(22) Date of filing: 25.09.2018
(51) Int. Cl.: D21C 9/14, D21C 11/00, D21C 11/06

(54) **METHOD OF CONTROLLING THE CHEMICAL BALANCE OF A PULP MILL**
VERFAHREN ZUR STEUERUNG DER CHEMIKALIENBILANZ EINER ZELLSTOFFFABRIK
PROCÉDÉ DE RÉGULATION DE L'ÉQUILIBRE CHIMIQUE D'UNE USINE DE PÂTE À PAPIER

(30) Priority: 25.09.2017 FI 20175852
(43) Date of publication of application: 05.08.2020
(73) Proprietor: Andritz Oy, 00180 Helsinki (FI)
(72) Inventor: PEHU-LEHTONEN, Lauri, 00180 Helsinki (FI)
(74) Representative: Espatent Oy
(86) International application number: PCT/FI2018/050691
(87) International publication number: WO 2019/058032

(56) References cited:
- WO-A1-2009/079746
- CA-A1- 2 219 550
- US-A- 2 881 052
- US-A- 4 081 520
- US-A- 4 508 593
- US-A- 4 938 943
- US-A- 4 961 918
- US-A- 5 300 191

## Description

The invention relates to a method for controlling the chemical balance at a sulfate pulp mill that produces at least pulp bleached with chlorine dioxide (ClO₂) and has a chlorine dioxide plant.

The pulp mill's current chemical recovery arrangement covers the recovery and regeneration of cooking chemicals, primarily sodium hydroxide (NaOH) and sodium sulfide (Na₂S). In spite of this, pulp mills produce significant quantities of side streams that may be harmful to the environment yet also contain significant quantities of valuable sodium and sulfur, which are lost along with these streams.

These side streams are typically created when the introduction of sulfurous chemicals into the process from the outside exceeds the amount of sulfur needed for the cooking. This leads to a situation where the pulp mill has a surplus of sulfur, which must be discharged from the process. Typically, the surplus sulfur is discharged as sodium sulfate (Na₂SO₄) into a nearby water system, which has been the most acceptable method of returning the sulfur waste flow back into nature. For example, most (80-90%) of the fly ash of the recovery boiler is sodium sulfate. Conventionally, fly ash dumped from the chemical recovery cycle has been dissolved in water or condensate, and the solution has been conveyed to the wastewater treatment plant. Due to increasingly strict environmental regulations, sodium sulfate emissions must be greatly reduced because they cause damage to inland water systems.

Pulp mills use chlorine dioxide for bleaching chemical pulp. There are several commercial chlorine dioxide production processes, which are known per se. All of the processes use sodium chlorate (NaClO₃) as a raw material. The process also requires an acid and a reducing agent. Typical current processes include the R8 process and R10 process. Their reducing agent is methanol (CH₃OH) and their acid is sulfuric acid (H₂SO₄). As such, the chlorine dioxide plant is one of the main consumers of sulfuric acid and methanol at pulp mills. In addition, these chemicals are brought in from outside the mill due to their strict quality requirements.

The chlorine dioxide plant produces by-products that contain sulfur and sodium. The R8 process produces an acid salt, sodium sesquisulfate (Na₃H(SO₄)₂), and the R10 process produces sodium sulfate, which can often be brought into the chemical recovery cycle or are dissolved into wastewater. In the latter case, the sulfate does not burden the mill's S/Na balance, but it increases the mill's wastewater emissions. However, a perfect recovery cycle of these chemicals has not been achieved, and therefore the surpluses of chemicals, mainly sulfur, formed by their introduction into the process, must be discharged in order to maintain chemical balance in the process.

US 4 961 918 discloses a method for production of chlorine dioxide, wherein purified sulphuric acid and methanol from the pulp mill process are used.

A purpose of the present invention is to provide a method where the introduction of chemicals into the pulp mill's processes can be significantly reduced or completely discontinued, thereby also minimising the need to discharge chemicals from the process.

The method of the present invention is defined in the current claim 1.

The new method forms additional chemical recovery cycles for sulfur and/or methanol. These additional chemical recovery cycles regenerate and produce internally purified chemicals that are of commercial quality and thus fulfil the high quality requirements set for e.g. the chlorine dioxide plant's raw materials, methanol and sulfuric acid. The introduction of chemicals from outside the process can be reduced, thereby reducing the amount of surplus sulfur at the pulp mill, the costs incurred by purchased chemicals and the use of fossil-based chemicals.

In the new method, the chemical balance is optimised and adjusted at the sulfate pulp mill, which produces at least pulp bleached with chlorine dioxide and has a chlorine dioxide plant using at least chlorate, methanol and sulfuric acid for producing chlorine dioxide. The method comprises at least the following steps:
a). gases from a concentrated non-condensable gas (CNCG) system of the mill are incinerated in order to form a gas containing sulfur dioxide (SO₂), which is treated to produce concentrated sulfuric acid, and
b). raw methanol from mill processes is purified to produce methanol, and
c). side streams containing sodium compounds and/or sulfur compounds produced by mill processes are used as make-up chemicals, wherein the production of chlorine dioxide uses sulfuric acid produced in step a) and methanol purified in step b), so that the sulfuric acid concentration is 94-99%, preferably 95-98%, and in step c) sesquisulfate or sodium sulfate produced during the production of chlorine dioxide is used.

Chlorine dioxide is produced from sodium chlorate, sulfuric acid and methanol in a manner known per se, for example using processes under the product name R8 or R10. Such processes are described in, for example, US patents 4081520 and 5066477. In the new method, the methanol and sulfuric acid used in the production of chlorine dioxide are produced at the pulp mill internally by producing commercial-grade methanol and sulfuric acid in new chemical recovery cycles.

In one embodiment of the new method, sulfuric acid produced in step a) is also used in at least one of the following processes: tall oil production, lignin recovery from black liquor, pulp bleaching, pH adjustment in the mill's chemical recovery process, microcrystalline cellulose (MCC) production. Sulfuric acid is also needed for certain methanol purification processes, for which acid produced internally is available. In one preferred embodiment, step b) uses sulfuric acid produced in step a), if the raw methanol is acidified in the methanol purification process. The use of sulfuric acid produced internally reduces the input of outside-sourced sulfur into the chemical recovery cycle. This also reduces the need to dump large amounts of ash of the recovery boiler from the chemical recovery cycle.

In one embodiment of the method, step c) also uses at least one of the following side streams: ash from the recovery boiler, sesquisulfate or sodium sulfate from chlorine dioxide production, waste acid from the chlorine dioxide plant, alkaline filtrates, such as EOP bleaching stage filtrate, from the pulp bleaching plant, which stage is an alkaline extraction stage of bleaching using sodium hydroxide, oxygen and peroxide. When internal material flows are used as make-up chemicals, this significantly reduces the amount of waste flows containing them from the mill. Furthermore, it helps avoid the loss of valuable chemicals that previously had to be discharged as waste in order to maintain the chemical balance.

When the dumping of ash from the recovery boiler decreases or perhaps even stops completely, the chlorine and potassium contained by the ash strive to accumulate in the chemical recovery cycle, which may cause fouling, plugging and corrosion of the recovery boiler's heating surfaces, for example, when the chlorine and potassium concentrations in black liquor reach a certain level. Therefore, it is preferable to reduce the amounts of chlorine and potassium in the ash through appropriate treatment before recirculating the ash into the process. Such treatments include ash leaching and crystallisation, for example. Ash leaching also uses sulfuric acid.

In one embodiment of the method, the sulfuric acid produced in step a) and the methanol purified in step b) constitute at least 70%, preferably 80%, of the need for these chemicals in chlorine dioxide production.

In one embodiment of the method, the purified methanol produced in step b) is also used as nitrogen-free fuel in order to reduce nitrous oxide emissions (NOₓ), thus reducing the use of fossil fuels and fossil-based chemicals at the pulp mill. Methanol produced internally can be used as a fuel for, for example, the recovery boiler and lime kiln and as a support fuel at the sulfuric acid plant.

For internal production of process chemicals at the mill, it is vital that the chemicals are of commercial quality so that they can replace purchased chemicals. Chlorine dioxide production is one process that has tight requirements for the purity of the input chemicals. However, purchased sulfuric acid and fossil-based methanol can be replaced with products produced at the pulp mill when sulfuric acid is produced from the mill's non-condensable gases and methanol is produced from the raw methanol generated by the mill's pulp production process. In that case, the only purchased chemical in chlorine dioxide production is sodium chlorate.

Sulfuric acid is produced preferably with the following method. The pulp mill's concentrated non-condensable gases are oxidised into sulfur dioxide in an incinerator. The gas is treated further in the presence of oxygen and a catalyst (such as V2O5) in order to oxidise the sulfur dioxide into sulfur trioxide (SO₃). The sulfur trioxide gas is condensed into the water vapour contained by the gas in order to form sulfuric acid. The sulfuric acid is cooled to storage temperature, and the excess water vapour is conveyed to flue gas treatment. For example, Haldor Topsoe's Wet gas Sulfuric Acid (WSA) process is one such sulfuric acid production process. The final product of the process is concentrated commercial-grade sulfuric acid, because the liquefaction of the acid is based a hundred per cent on condensation. Therefore, concentrated commercial-grade sulfuric acid can be produced in-mill, significantly reducing the amount and costs of purchased chemicals. The concentration of sulfuric acid is 94-99%, preferably 95-98%.

Because the sulfuric acid production process works above the dew point of sulfuric acid, from incineration all the way to the condenser, the metal hardware can be constructed from standard materials, such as carbon steel, without corrosion issues in the system.

Sulfuric acid production at the mill reduces the dumping of fly ash by an amount corresponding to the decrease in surplus sulfur. Most often, the discharging of surplus sulfur from the process can be completely eliminated, which also helps avoid large sodium losses. This, in turn, reduces the amount of make-up sodium hydroxide required and the associated costs.

Methanol is obtained during the sulfate process at pulp mills as a side product. The raw methanol obtained contains impurities, such as sulfur compounds, ethanol, ammonium and turpentine. Raw methanol is primarily obtained from condensates of black liquor evaporation. It is also obtained from other items at the pulp mill, such as the vapours and condensates of the cooking stage.

Raw methanol can be purified of sulfur compounds and other impurities to produce commercial-grade methanol, for example with the process presented in publication WO/2015/053704. The methanol content of raw methanol is typically at least 65 wt%. In such cases, the raw methanol is washed with non-polar organic solvent, which treatment can be carried out at the pulp mill, and the purified methanol can be used in mill processes. The solvent can be regenerated and reused. Non-polar organic solvent is typically a solvent containing at least 50 wt% of C8-C20 alkanes and/or cycloalkanes or containing at least 50 wt% of triglycerides that are dissolved at temperatures under 40 °C. The solvent can be, for example, white mineral oil, mineral oil, white oil, paraffin oil or a mixture thereof. Before the solvent treatment, the raw methanol is acidified, typically with sulfuric acid, and the resulting precipitate, mainly ammonium sulfate ((NH)₂SO₄), is extracted from the methanol. Ammonium sulfate can be utilised at a biological wastewater treatment plant. Turpentine can also be discharged and sulfur impurities distilled off before the solvent treatment. Pre-treated raw methanol is typically diluted with water before the solvent treatment.

In place of the methanol purification process described above, other processes can be used to produce pure methanol.

Commercial-grade methanol must meet the quality requirements set by the IMPCA (International Methanol Producers & Consumers Association, IMPCA Reference Specification), which means that the methanol is essentially free of sulfur and nitrogen.

In the new method, the recirculations of methanol and sulfur form additional chemical recovery cycles. The sulfuric acid plant is an additional chemical recovery cycle where sulfur in concentrated non-condensable gases is recovered and regenerated into sulfuric acid. This creates a new kind of sulfur cycle in connection with the existing chemical recovery cycle. Methanol, in turn, is recovered from the plant's condensates, for example, and used in purified form for chlorine dioxide production, which forms a new type of methanol cycle. This sulfur cycle and methanol cycle form new type of chemical recovery cycles in connection with chlorine dioxide production.

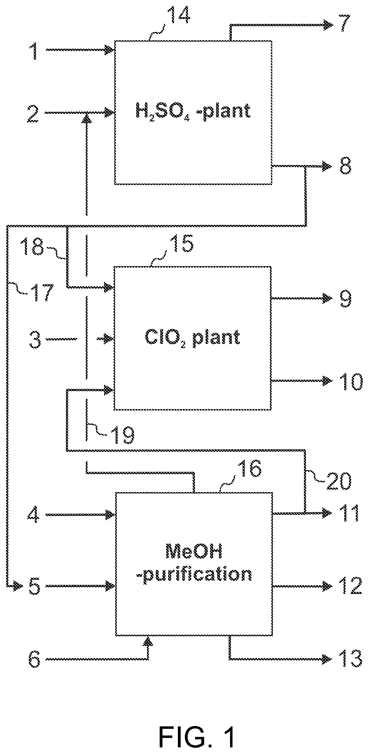

The new method is explained in more detail with reference to the figure provided, which diagrammatically presents one embodiment for its implementation.

A sulfuric acid plant 14 produces sulfuric acid from the mill's concentrated non-condensable gases (CNCG) 2, which are oxidised into sulfur dioxide in an incinerator. The incineration requires support fuel 1, which may be methanol, as well as oxygen. The energy produced by the incinerator is recovered as steam 7, typically as intermediate-pressure steam (10-45 bar). After the incineration, the gas is treated further in the presence of oxygen and a catalyst (such as V₂O₅) to oxidize the sulfur dioxide into sulfur trioxide. The sulfur trioxide gas is condensed into the water vapour contained by the gas in order to form sulfuric acid. The sulfuric acid is cooled to storage temperature, and the excess water vapour is conveyed to flue gas treatment. The final product of the process is concentrated commercial-grade sulfuric acid, because the liquefaction of the acid is based 100% on condensation. The concentration of sulfuric acid is 94-99%, preferably 95-98%.

The sulfuric acid produced can be used according to the new method in chlorine dioxide production at a plant 15, to which the sulfuric acid is conveyed through line 18.

Raw methanol produced at the pulp mill is purified in a process that can produce the clean methanol required by the chlorine dioxide plant. The process comprises the following stages at a plant 16. Raw methanol 4, which contains sulfur compounds and ammonium compounds, is oxidised with sulfuric acid 5, which forms precipitate containing ammonium sulfate 13. This can be utilised at a biological wastewater treatment plant as a nutrient source for microbes. The sulfuric acid is preferably produced at the mill as described above and brought from the sulfuric acid plant 14 through line 17. During the acidification, turpentine 12 can be extracted from the raw methanol through decantation. After the acidification and precipitate removal, the methanol is distilled, and the distilled methanol is diluted with water 6. Turpentine can be discharged from the diluted methanol before the methanol is washed with a non-polar organic solvent. Methanol, ethanol and acetone can still be discharged from the washed methanol through distillation. This enables the production of the clean methanol 11 required for chlorine dioxide production by in-mill sources.

Sulfuric gases separated during methanol purification are conveyed to sulfuric acid production through line 19.

The sulfuric acid and methanol produced in-mill are used at the chlorine dioxide plant 15, to which the sulfuric acid is conveyed through line 18 and methanol through line 20. Chlorine dioxide 9 is produced in a manner known per se, using e.g. processes R8 or R10, from sodium chlorate 3. The chlorine dioxide plant produces by-products that contain sulfur and sodium 10. The R8 process also generates an acid salt, sodium sesquisulfate 10, and the R10 process generates sodium sulfate 10, which can be brought into the chemical recovery cycle as make-up chemicals.

Sulfuric acid 8 can also be utilised, for example, at the mill's possible tall oil plant, for lignin recovery from black liquor, for pulp bleaching. Purified methanol 11 can also be used as support fuel 1 for the sulfuric acid plant and as fuel in the recovery boiler and/or lime kiln.

## Claims

1. A method for controlling the chemical balance at a sulfate pulp mill, producing at least pulp bleached with chlorine dioxide and which has a chlorine dioxide plant using at least chlorate, methanol and sulfuric acid to produce chlorine dioxide, the method comprising at least the following steps:
a). gases from the mill's concentrated non-condensable gas system are incinerated in order to form a gas containing sulfur dioxide, which is treated to produce concentrated sulfuric acid, and
b). raw methanol from mill processes is purified to produce methanol, and
c). side streams containing sodium compounds and/or sulfur compounds produced by mill processes are used as make-up chemicals, wherein
the production of chlorine dioxide uses the sulfuric acid produced in step a) and methanol purified in step b), so that the sulfuric acid concentration is 94-99%, preferably 95-98%, and in step c) sesquisulfate or sodium sulfate produced during the production of chlorine dioxide is used.

2. The method according to claim 1, **characterised in that** sulfuric acid produced in step a) is also used in at least one of the following processes: tall oil production, lignin recovery from black liquor, pulp bleaching, pH adjustment in the mill's chemical recovery process, microcrystalline cellulose production.

3. The method according to claim 1 or 2, **characterised in that** step c) also uses at least one of the following side streams: ash from the recovery boiler, sesquisulfate or sodium sulfate from chlorine dioxide production, waste acid from the chlorine dioxide plant, alkaline filtrates from a pulp bleaching plant.

4. The method according to claim 1, 2 or 3, **characterised in that** the sulfuric acid produced in step a) and the methanol produced in step b) constitute at least 70%, preferably 80%, of the need for these chemicals in the chlorine dioxide production.

5. The method according to any one of the preceding claims, **characterised in that** the purified methanol produced in step b) is also used as nitrogen-free fuel in order to reduce nitrous oxide emissions.

6. The method according to any one of the preceding claims, **characterised in that** in step a), the gas containing sulfur dioxide is treated in the presence of oxygen and a catalyst in order to oxidise the sulfur dioxide into sulfur trioxide, which is condensed into water vapour contained by the gas to produce concentrated sulfuric acid.

## Patentansprüche

1. Verfahren zur Steuerung des chemischen Gleichgewichts in einem Sulfatzellstoffwerk, das mindestens Zellstoff herstellt, der mit Chlordioxid gebleicht ist, und das eine Chlordioxidanlage aufweist, die mindestens Chlorat, Methanol und Schwefelsäure zur Herstellung von Chlordioxid verwendet, wobei das Verfahren mindestens die folgenden Schritte umfasst:
a) Gase aus dem konzentrierten nicht kondensierbaren Gassystem des Werks werden verbrannt, um ein Schwefeldioxid enthaltendes Gas zu bilden, das zur Herstellung konzentrierter Schwefelsäure behandelt wird, und
b) Rohmethanol aus Werksprozessen wird gereinigt, um Methanol herzustellen, und
c) Nebenströme, die Natriumverbindungen und / oder Schwefelverbindungen enthalten, die durch Werksprozesse hergestellt werden, werden als Zugabechemikalien verwendet, wobei die Herstellung von Chlordioxid die in Schritt a) hergestellte Schwefelsäure und das in Schritt b) gereinigte Methanol verwendet, so dass die Schwefelsäurekonzentration 94-99 %, bevorzugt 95-98 %, beträgt, und in Schritt c) Sesquisulfat oder Natriumsulfat verwendet wird, hergestellt bei der Herstellung von Chlordioxid.

2. Verfahren nach Anspruch 1, **dadurch gekennzeichnet, dass** die in Schritt a) hergestellte Schwefelsäure auch in mindestens einem der folgenden Prozesse verwendet wird:
Tallölherstellung, Ligninrückgewinnung aus Schwarzlauge, Zellstoffbleiche, pH-Einstellung in dem chemischen Rückgewinnungsprozess des Werks, Herstellung von mikrokristalliner Cellulose.

3. Verfahren nach Anspruch 1 oder 2, **dadurch gekennzeichnet, dass** Schritt c) auch mindestens einen der folgenden Nebenströme verwendet: Asche aus dem Rückgewinnungskessel, Sesquisulfat oder Natriumsulfat aus Chlordioxidherstellung, Abfallsäure aus der Chlordioxidanlage, alkalische Filtrate aus einer Zellstoffbleichanlage.

4. Verfahren nach Anspruch 1, 2 oder 3, **dadurch gekennzeichnet, dass** die in Schritt a) hergestellte Schwefelsäure und das in Schritt b) hergestellte Methanol mindestens 70 %, bevorzugt 80 %, des Bedarfs an diesen Chemikalien in der Chlordioxidherstellung ausmachen.

5. Verfahren nach einem der vorhergehenden Ansprüche, **dadurch gekennzeichnet, dass** das in Schritt b) hergestellte gereinigte Methanol auch als stickstofffreier Brennstoff verwendet wird, um Stickoxidemissionen zu reduzieren.

6. Verfahren nach einem der vorhergehenden Ansprüche, **dadurch gekennzeichnet, dass** in Schritt a) das schwefeldioxidhaltige Gas in der Gegenwart von Sauerstoff und einem Katalysator behandelt wird, um das Schwefeldioxid zu Schwefeltrioxid zu oxidieren, das zu dem in dem Gas enthaltenen Wasserdampf kondensiert wird, um konzentrierte Schwefelsäure herzustellen.

## Revendications

1. Un procédé pour réguler l'équilibre chimique dans une usine de fabrication de pâte à papier au sulfate, produisant au moins de la pâte à papier blanchie au dioxyde de chlore et qui comprend une installation de production de dioxyde de chlore utilisant au moins du chlorate, du méthanol et de l'acide sulfurique pour produire du dioxyde de chlore, le procédé comprenant au moins les étapes suivantes :
a). les gaz provenant du système de gaz non condensables concentrés de l'usine sont incinérés afin de former un gaz contenant du dioxyde de soufre, qui est traité pour produire de l'acide sulfurique concentré, et
b). le méthanol brut provenant des processus de l'usine est purifié pour produire du méthanol, et
c). les flux secondaires contenant des composés de sodium et/ou des composés de soufre produits par les processus de l'usine sont utilisés comme produits chimiques d'appoint,
la production de dioxyde de chlore utilise l'acide sulfurique produit à l'étape a) et le méthanol purifié à l'étape b), de manière à obtenir une concentration en acide sulfurique de 94 à 99 %, de préférence de 95 à 98 %, et à l'étape c), le sesquisulfate ou le sulfate de sodium produit pendant la production de dioxyde de chlore est utilisé.

2. Le procédé selon la revendication 1, **caractérisé en ce que** l'acide sulfurique produit à l'étape a) est également utilisé dans au moins l'un des procédés suivants : production de tall oil, récupération de lignine à partir de liqueur noire, blanchiment de pâte à papier, ajustement du pH dans le procédé de récupération chimique de l'usine, production de cellulose microcristalline.

3. Le procédé selon la revendication 1 ou la revendication 2, **caractérisé en ce que** l'étape c) utilise également au moins l'un des flux secondaires suivants :
cendres provenant de la chaudière de récupération, sesquisulfate ou sulfate de sodium provenant de la production de dioxyde de chlore, acide résiduaire provenant de l'installation de dioxyde de chlore, filtrats alcalins provenant d'une installation de blanchiment de la pâte à papier.

4. Le procédé selon la revendication 1, 2 ou 3, **caractérisé en ce que** l'acide sulfurique produit à l'étape a) et le méthanol produit à l'étape b) constituent au moins 70 %, de préférence 80 %, des besoins en ces produits chimiques dans la production de dioxyde de chlore.

5. Le procédé selon l'une quelconque des revendications précédentes, **caractérisé en ce que** le méthanol purifié produit à l'étape b) est également utilisé comme combustible exempt d'azote afin de réduire les émissions d'oxyde nitreux.

6. Le procédé selon l'une quelconque des revendications précédentes, **caractérisé en ce que**, à l'étape a), le gaz contenant du dioxyde de soufre est traité en présence d'oxygène et d'un catalyseur afin d'oxyder le dioxyde de soufre en trioxyde de soufre, lequel est condensé en vapeur d'eau contenue dans le gaz pour produire de l'acide sulfurique concentré.
